# EUROPEAN PATENT APPLICATION

(11) **EP 2 671 887 A1**
(43) Date of publication of application: **11.12.2013**
(21) Application number: 12742042.0
(22) Date of filing: 31.01.2012
(51) Int. Cl.: C07H 17/08, A61K 31/7048, A61P 31/04

(54) **NOVEL MACROLIDE DERIVATIVE**

(30) Priority: 31.01.2011 JP 2011018842
(71) Applicant: Meiji Seika Pharma Co., Ltd., Tokyo 104-8002 (JP)
(72) Inventor: KURIHARA, Kenichi, Yokohama-shi Kanagawa 222-8567 (JP); MITOMI, Masaaki, Yokohama-shi Kanagawa 222-8567 (JP)
(74) Representative: KATZAROV S.A.
(86) International application number: PCT/JP2012/052153
(87) International publication number: WO 2012/105562

(57) **Abstract**

The inventors of the present invention have succeeded in acquiring a compound having excellent antibacterial activities against pathogens of respiratory tract infections in animals through the use of josamycin as a lead scaffold.

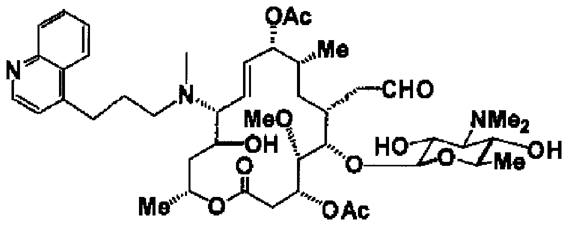

## Description

### TECHNICAL FIELD

The present invention relates to a novel macrolide derivative effective as a therapeutic drug for bacterial infections in animals.

### BACKGROUND ART

Among macrolides as animal antibacterial drugs, erythromycin, tylosin, and tilmicosin are mainly used as injection preparations or oral preparations for treating bacterial respiratory tract infections in cattle and swine. Further, natural products classified as leucomycin-type 16-membered ring macrolides, such as josamycin, kitasamycin, and spiramycin, do not have indications as therapeutic drugs for respiratory tract infections in cattle, but are used as oral antibacterial agents for treating bacterial respiratory tract infections in swine.

Meanwhile, among antibacterial drugs for respiratory tract infections in humans, macrolides that are most frequently used in clinical practice at present are clarithromycin, which is obtained by 6-*O*-methylation of erythromycin, and azithromycin, which is an azalide-type 15-membered ring macrolide obtained by introduction of a nitrogen atom into a lactone ring of erythromycin.

Tulathromycin is a recently developed azalide-type 15-membered ring macrolide exclusively for animals. Tulathromycin was developed as an animal drug for treating and preventing bacterial respiratory tract diseases in cattle and swine, approved as a drug for treating and preventing bacterial respiratory tract diseases in cattle and swine in the EU in 2003 and in the US in 2005, and then approved in Australia, Canada, Asian countries, and the like. In Japan, tulathromycin is being filed for approval as an injection preparation for swine.

Meanwhile, tylosin and tilmicosin are antibacterial agents exclusively for animals, which are not used for humans. Tilmicosin is a macrolide which has improved antibacterial activities against gram-negative bacteria and is synthesized from tylosin, and has indications for pneumonia including pneumonic pasteurellosis and mycoplasmal pneumonia in cattle and swine.

On the other hand, the leucomycin-type 16-membered ring macrolides each have only an indication for mycoplasmal pneumonia in swine. Among the leucomycin-type 16-membered ring macrolides, for example, josamycin is a drug used in humans as well, and is effective for gram-positive bacteria clinically problematic in respiratory tract infections in humans. As pathogens problematic in respiratory tract infections in livestock animals, there are given gram-positive bacteria and mycoplasma, and as other representative examples, gram-negative bacteria such as *Mannheimia haemolytica*, *Histophilus somni*, and *Pasteurella multocida* in cattle and *Actinobacillus pleuropneumoniae*, *Haemophilus parasuis*, and *Pasteurella multocida* in swine. The biggest possible reason why the leucomycin-type macrolide has a limited indication on a livestock site is that the macrolide has weak effects against those gram-negative bacteria. Therefore, creation of such an antibacterial drug exclusively for animals having the scaffold as to solve those problems could contribute to inhibition of emergence of resistant bacteria, thereby providing certain effects.

In such circumstances, the inventors of the present invention found that a derivative of midecamycin, a 16-membered ring macrolide, modified at the C-12 and C-13 positions had excellent antibacterial activities (WO2002/064607).

### SUMMARY OF INVENTION

### TECHNICAL PROBLEM

An object of the present invention is to provide a novel macrolide derivative effective for gram-negative bacteria.

### SOLUTION TO PROBLEM

The inventors of the present invention have discovered that a derivative of midecamycin modified at the C-12 and C-13 positions has excellent antibacterial activities (WO2002/064607), and have had interest in josamycin because there is no finding on modification at the C-12 and C-13 positions of josamycin, which partially differs from midecamycin in lactone ring structure. In view of the foregoing, the inventors have synthesized a derivative using josamycin as a lead scaffold. As a result, the inventors have found that the derivative exhibits extremely strong antibacterial actions against pathogens of bacterial respiratory tract infections problematic in livestock animals such as cattle and swine. That is, the inventors have revealed that a compound of the following formula (I) gives a minimum inhibitory concentration (MIC: minimum drug concentration completely inhibiting the growth of a test bacterial strain) corresponding to an activity enhanced approximately 4-fold or more as compared to tulathromycin as the latest animal macrolide and approximately 8-fold as compared to josamycin, and have found that the compound has extremely strong antibacterial activities against main pathogens of bacterial respiratory tract infections in livestock animals such as cattle and swine.

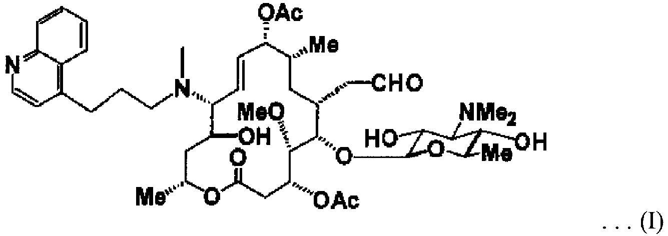

The present invention relates to a compound exhibiting excellent antibacterial activities against bacterial infections in animals, and to applications of the compound. More specifically, the present invention provides descriptions about the following items.

[1] A compound, which is represented by the formula (I), or a pharmacologically acceptable salt thereof.

[2] A drug, including as an active ingredient the compound or the pharmacologically acceptable salt thereof according to the item 1.

[3] The drug according to the item 2, which is used as an antibacterial drug.

[4] A pharmaceutical composition, including as an active ingredient the compound or the pharmacologically acceptable salt thereof according to the item 1, and an additive for formulation.

[5] An animal drug, including as an active ingredient the compound according to the item 1.

[6] An animal antibacterial drug, including as an active ingredient the compound according to the item 1.

### ADVANTAGEOUS EFFECTS OF INVENTION

According to the present invention, there is provided the compound which not only is effective for gram-positive bacteria, mycoplasma, chlamydia, and rickettsia in the same manner as general macrolides, but also exhibits strong antibacterial actions against gram-negative bacteria problematic in infections in animals, in particular. Further, the compound of the present invention can exhibit excellent antibacterial effects against pathogens of respiratory tract infections in animals such as cattle and swine. In addition, the use of the compound of the present invention allows infections in animals, such as lung infection, mastitis, bacteremia, septicemia, and diarrhea, to be treated or prevented effectively.

### DESCRIPTION OF EMBODIMENTS

A compound according to the present invention is effective for bacterial pathogens as exemplified below. Bacterial pathogens of swine diseases, such as *Bacillus anthracis*, *Brucella suis*, *Clostridium chauvoei*, *Leptospira* spp., *Salmonella* serovar *Dublin*, *S. Enteritidis*, *S. Typhimurium*, *S. Choleraesuis, Francisella tularensis, F holarctia*, *F. mediasiatica*, *F. novicida*, *Bordetella bronchiseptica* and toxigenic *Pasteurella multocida, Erysipelothrix rhusiopathiae, Brachyspira hyodysenteriae, Staphylococcus hyicus*, *Lawsonia intracellularis*, verotoxigenic *Escherichia coli* (VTEC), *Actinobacillus equuli, A. pleuropneumoniae, A. susi, Arcanobacterium pyogenes, Clostridium perfringens* type C, *Actinobacillus pleuropneumoniae*, *Mycobacterium avium-intracellulare* complex, enterotoxigenic *Escherichia coli* (ETEC), attaching and effacing *Escherichia coli* (AEEC), *Pasteurella multocida* type B or E, *Mycoplasma hyopneumoniae*, *Streptococcus suis*, and *Haemophilus parasuis.*, Bacterial pathogens of bovine diseases, such as *Bacillus anthracis*, *Brucella abortus*, *B. canis*, *Mycobacterium bovis*, *Mycobacterium avium* subsp. *paratuberculosis*, *Clostridium chauvoei, Clostridium tetani*, *Leptospira*, *Salmonella* serovar *Dublin*, *S. Enteritidis*, *S. Typhimurium*, *S. Choleraesuis*, *Campylobacter fetus* subsp. *venerealis*, *C. fetus* subsp. *fetus*, *Clostridium septicum*, *C. sordellii*, *C. perfringens* (type A), *C*. *novyi* (type A), *Actinobacillus lignieresii, Clostridium perfringens, Corynebacterium renale, C. pilosum, C. cystitidis,* enterotoxigenic *Escherichia coli* (ETEC), verotoxigenic *Escherichia coli* (VTEC), attaching and effacing *Escherichia coli* (AEEC), *Pasteurella multocida*, *Mannheimia haemolytica*, *P. trehalosi*, *Mycoplasma mycoides* subsp. *mycoides* small colony (SC) type, *Clostridium botulinum* type C or D toxingenic bacterium, *Mycoplasma bovis*, *M. bovigenitalium*, *M. dispar*, *Ureaplasma diversum*, *Mycoplasma alkalescens*, *M. arginini*, *M. bovigenitalium, M. bovirhinis, M. bovis, M. californicum, M. canadense, Fusobacterium necrophorum, Moraxella bovis, Histophilus somni, Actinomyces bovis, Listeria monocytogenes,* and *Dermatophilus congolensis,* and bacterial pathogens of poultry diseases, such as *Pasteulella multocida, Salmonella Pu-llorum* (*Salmonella enterica* subsp. *enterica* serovar *Gallinarum* biovar *Pullorum*) (pullorum disease), *Salmonella Gallinarum* (*Salmonella enterica* subsp. *enterica* serovar, *Gallinarum* biovar *Gallinarum* (fowl typhoid), *S. enterica*, *S. Typhimurium*, *S. Avibacterium*, *Haemophilus paragallinarum*, *Clostridium perfringens* type A or C, *Escherichia coli, Staphylococcus aureus, S. hyicus,* and *Clostridium botulinum* type C toxingenic bacterium.

Further, the compound according to the present invention is effective for bacterial diseases as exemplified below problematic in infections in animals, in particular.

In swine, the compound is effective for swine anthrax, swine brucellosis, swine blackleg, swine leptospirosis, swine Weil's disease, swine salmonellosis, swine tularemia, atrophic rhinitis in swine, swine erysipelas, swine exudative epidermitis (exudative dermatitis, *Staphylococcus hyicus* infection), swine proliferative enteritis, swine edema disease, swine actinobacillosis, *Arcanobacterium pyogenes* infection in swine, swine necrotic enteritis, swine pleuropneumonia, mycobacterial infection in swine, swine colibacillosis, swine hemorrhagic septicemia, swine pasteurellosis (pneumonic pasteurellosis), streptococcal infection in swine, *Haemophilus parasuis* infection in swine (Glasser's disease), *Actinomyces pyogenes* infection in swine, swine yersiniosis, *Bacteroides* infection in swine, swine brucellosis, swine dysentery, swine enterotoxemia, streptococcal infection in swine, swine staphylococcosis, *Pseudomonas aeruginosa* infection in swine, swine eperhythrozoonosis, chlamydial infection in swine, mycoplasmal pneumonia in swine, swine mycoplasmal arthritis, and the like.

In cattle, the compound is effective for bovine anthrax, bovine brucellosis, bovine tuberculosis, bovine Johne's disease, bovine blackleg, bovine tetanus, leptospirosis (bovine leptospirosis), bovine nocardiosis, bovine enterotoxemia, bovine tuberculosis, salmonellosis (bovine salmonellosis), bovine genital campylobacteriosis, bovine malignent edema, bovine actinobacillosis, bovine necrotic enteritis, *Corynebacterium* urinary tract infection in cattle, bovine colibacillosis, bovine hemorrhagic septicemia, *Pasteurella* (*Mannheimia*) infection in cattle, mastitis, coliform mastitis, *Haemophilus somnus* infection in cattle, contagious bovine pleuropneumonia, bovine botulism, bovine mycoplasmal pneumonia, bovine mycoplasmal mastitis, bovine liver abscess, infectious bovine keratoconjunctivitis, bovine cystitis, bovine pyelonephritis, bovine listeriosis, bovine necrobacillosis, bovine actinomycosis, bovine dermatophilosis, contagious bovine pleuropneumonia, abortion and infertility in cattle, sporadic bovine encephalomyelitis, bovine polyarthritis, tick fever, ehrlichiosis, bovine petechial fever, coxiellosis, anaplasmosis, bovine eperhythrozoonosis, and the like.

In horses, the compound is effective for equine glanders, equine melioidosis, equine tetanus, equine paratyphoid, equine *Klebsiella* infection, contagious equine metritis, *Rhodococcus equi* infection, equine strangles, chlamydial infection in horses and Potomac horse fever. In ovine and caprine diseases, the compound is effective for brucellosis, ovine dysentery, pseudotuberculosis, nonsuppurative polyarthritis, *Erysipelothrix rhusiopathiae* infection in sheep, clostridial infection in sheep, contagious ovine digital dermatitis, tularemia, heartwater, contagious ophthalmia, enzootic abortion in ewes, ovine polyarthritis, transmissible serositis, contagious agalactia, contagious caprine pleuropneumonia, and the like.

In dogs and cats, the compound is effective for canine leptospirosis, canine Lyme disease, canine brucellosis, canine campylobacteriosis, *Bordetella* infection in dogs, anaerobic infection in dogs and cats, feline leptospirosis, feline tuberculosis, canine ehrlichiosis, salmon poisoning, cat-scratch disease, feline hemobartonellosis, chlamydial infection in cats, and mycoplasmosis in dogs and cats. In poultry, fowl cholera, anatipestifer infection in birds, avian paratyphoid, *Salmonella arizonae* infection, avian staphylococcosis, *Histophilus somni* infection, actinomycosis, listeriosis, dermatophilosis, digital papillomatosis, avian pasteurellosis, *Salmonella* infection in poultry, salmonellosis (avian paratyphoid), avian tuberculosis, infectious coryza, *Erysipelothrix rhusiopathiae* infection in birds, avian campylobacteriosis, clostridial disease in poultry, avian necrotic enteritis, avian colibacillosis, avian staphylococcosis, avian botulism, streptococcal infection in birds, avian spirochetosis, avain rhinotracheitis, aegyptianellosis, avian chlamydiosis, avian mycoplasmosis, mycoplasmal synovitis in poultry, and the like.

In fish, the compound is effective for vibriosis, non-motile aeromonas infection, motile aeromonas septicemia, bacterial kidney disease, edwardsiellosis, coldwater disease, columnaris disease, pseudotuberculosis, pasteurellosis, red mouth disease, nocardiosis, mycobacterial infection in fish, *Pseudomonas* infection, bacterial gill disease, streptococcal infection, and the like.

In addition, the compound not only may be used as a therapeutic drug for the diseases exemplified above but also may be used as a therapeutic drug based particular effects other than antibacterial actions, such as an immunostimulatory action inherent in a macrolide and an effect against a biofilm.

The compound represented by the formula (I) or the salt thereof of the present invention may be produced, for example, by a method to be described later or a method similar thereto. The details thereof are described.

First, a description is made of a production method for 9-O-acetyl-12-azide-12,13-dihydro-13-hydroxyjosamycin 18-dimethylacetal as a compound of Example 1. Through the use of josamycin as a starting material, a hydroxy group at the C-9 position was subjected to selective modification with an acyl group, and a formyl group at the C-18 position was then subjected to modification with an acetal-type protective group. The modification of the hydroxy group at the C-9 position with the acyl group proceeds through a reaction with an acid halide in the presence of pyridine in a methylene chloride solvent. A solvent in this reaction may be methylene chloride or any other aprotic solvent such as chloroform, benzene, toluene, or xylene. A base is preferably an organic base such as pyridine, and is recommended to be used in 1 to 10 equivalents. As an acylation reagent, it is recommended to use 1 to 5 equivalents of acetyl chloride. The reaction proceeds in good yield in the range of 20°C to 60°C, and the reaction time is 1 hour to 24 hours. The subsequent modification of the formyl group at the C-18 position with the acetal-type protective group is carried out in the presence of an organic acid in a mixed solvent of methyl orthoformate and methanol, and thus proceeds in good yield. An acid to be used may be an organic acid such as p-toluenesulfonic acid or camphorsulfonic acid, but is preferably pyridinium p-toluenesulfonate (PPTS). Further, it is recommended to use, as a solvent, a 10-fold amount (V/W) to a 60-fold amount (V/W) of a mixed solution of equal amounts of methyl orthoformate and methanol serving as reagents as well. The reaction proceeds in good yield in the range of 20°C to 80°C, and the reaction time is 1 hour to 4 days.

Then, the compound obtained in the reaction described above was subjected to epoxidation at the C-12 and C-13 positions through a reaction with 3-chloroperoxybenzoic acid (m-CPBA) in chloroform, followed by selective reduction of the simultaneously oxidized dimethylamino group moiety with sodium dithionite, to thereby afford an epoxy product of interest. An epoxidation agent to be used in this reaction may be a peracid such as monoperoxyphthalic acid, trifluoroperacetic acid, or peracetic acid or peroxide such as dioxirane. It is preferably recommended to use 1 to 10 equivalents of m-CPBA. A solvent to be used in this reaction is preferably a halogenated solvent such as chloroform or methylene chloride. The reaction proceeds in good yield in the range of 0°C to 50°C, and the reaction time is 1 hour to 36 hours. In the subsequent selective reduction reaction, it is recommended to add a lower alcohol to the reaction solvent described above and then add an aqueous solution having dissolved therein a reducing agent such as sodium thiosulfate. It is preferably recommended to add ethanol and then use 1 to 4 equivalents of a 1 to 10% aqueous solution prepared by dissolving sodium dithionite. The reaction proceeds in good yield in the range of -15°C to 20°C, and the reaction time is 5 minutes to 1 hour.

Subsequently, the epoxy product obtained in the reaction described above was subjected to a reaction with sodium azide in the presence of ammonium chloride to afford the compound of Example 1. A solvent to be used in this reaction is preferably a mixed solvent of a lower alcohol such as methanol or ethanol and water. An additive to be used may be ammonium chloride or any other additive such as ammonium bromide or ammonium thiocyanate, and is recommended to be used in 1 to 10 equivalents. Sodium azide is used in 1 to 15 equivalents. The reaction proceeds in good yield in the range of 20°C to 100°C, and the reaction time is 1 hour to 48 hours.

Second, a description is made of a production method for 9-O-acetyl-12-amino-12,13-dihydro-13-hydroxyjosamycin 18-dimethylacetal as a compound of Example 2. A reduction reaction of the compound of Example 1 using triphenylphosphine afforded a product of interest. A solvent to be used in this reaction is preferably acetonitrile, THF, diethyl ether, or the like. A reagent to be used may be triphenylphosphine or any other trialkylphosphine such as trimethylphosphine or triethylphosphine, and is recommended to be used in 1 to 2 equivalents. The reaction proceeds in good yield in the range of 20°C to 100°C, and the reaction time is 1 hour to 48 hours.

Third, a description is made of a production method for 9-O-acetyl-12,13-dihydro-13-hydroxy-12-(N-methyl-N-(3-(quinolin-4-yl)propyl)aminoj osamycin 18-dimethylacetal as a compound of Example 3. To the compound of Example 2 was added 3-(quinolin-4-yl)propylcarbaldehyde in the presence of acetic acid, and the mixture was subjected to a reductive alkylation reaction to an amino group with sodium cyanoborohydride, to thereby afford the compound of interest. A reagent to be used in this reductive alkylation reaction is recommended to be used in 1 to 5 equivalents, and there may be used, as a solvent, a lower alcohol such as methanol or ethanol or any other solvent such as acetonitrile or methylene chloride. Acetic acid to be added is used in 1 to 15 equivalents. A reducing agent may be sodium acetoxyborohydride, picoline borane, or the like, and it is preferably recommended to use 1 to 5 equivalents of sodium cyanoborohydride. The reaction proceeds in good yield in the range of 20°C to 100°C, and the reaction time is 30 minutes to 24 hours.

Next, to the compound described above was added a formaldehyde solution in the presence of acetic acid, and a reductive alkylation reaction of the amino group with sodium cyanoborohydride was carried out. It is recommended that the reductive alkylation reaction in this reaction be carried out in the same manner as the method described above.

Fourth, a description is made of a production method for 9-O-acetyl-4'-demycarosyl-12,13-dihydro-13-hydroxy-12-(N-methyl-N-(3-(quinolin-4-yl)propyl)aminojosamycin of Example 4 (compound of formula (I)). The removal of the protective group at the C-18 position of the compound of Example 3 through a reaction with difluoroacetic acid in a mixed solvent of acetonitrile and water afforded the compound of interest. It is recommended to use, as a solvent to be used in this reaction, a 10-fold amount (g/ml) to a 300-fold amount (g/ml) of a mixed solution of equal amounts of acetonitrile and water. Further, monofluoroacetic acid, trifluoroacetic acid, acetic acid, or the like as well as difluoroacetic acid may be used, and is recommended to be used in 1 to 30 equivalents. The reaction proceeds in good yield in the range of 20°C to 50°C, and the reaction time is 12 hours to 4 days.

It should be noted that the present invention is by no means limited to the examples, and encompasses all of synthesis, production, extraction, and purification methods for the compounds by known means based on the properties of the compounds revealed by the present invention as well as modification means of the examples.

Hereinafter, a description is made of an evaluation method for the compound. The compound was measured for its in vitro antibacterial activity by a broth microdilution method in accordance with a CLSI method (formerly NCCLS method, M31-A2) (Performance Standards for Antimicrobial Disk and Dilution Susceptibility Tests for Bacteria Isolated from Animals; Approved Standard-Second Edition NCCLS M31-A2 Vol. 22 No. 6 2002). A medium used was BBL Mueller Hinton II broth supplemented with lysed horse blood and NAD. A test drug solution at each concentration level obtained by dissolving a test drug in ethanol and then diluting the resultant with the liquid medium described above was dispensed into a 96-well microplate, and a test bacterial strain was inoculated. After having been cultured in the presence of 5% CO₂ at 37°C for 20 to 24 hours, the test bacterial strain was visually observed for the presence or absence of its growth. A minimum drug concentration completely inhibiting the growth of the test bacterial strain was defined as a minimum inhibitory concentration (hereinafter, referred to as MIC).

A substance selected from the group consisting of the compound of the formula (I) and a pharmacologically acceptable salt and hydrate thereof, and a solvate thereof may be used without any treatment. In general, however, it is preferred to prepare and administer a composition including the substance as an active ingredient and one or two or more additives for formulation. The compound of the present invention may be administered to animals via any of oral and parenteral administration routes (e.g., intravenous injection, intramuscular injection, subcutaneous injection, intradermal injection, intraperitoneal administration, rectal administration, and transdermal administration). The compound of the present invention may be prepared as a composition in an appropriate form depending on its administration route. Specifically, the compound may be prepared mainly as a composition in any form including injection preparations for intravenous administration, for intramuscular administration, for subcutaneous administration, for intradermal administration, and for intraperitoneal administration, capsules, tablets, granules, powders, pills, fine granules, syrups, troche tablets, and other oral preparations, inhalants, rectal dosage forms, oleaginous suppositories, aqueous suppositories, lotions, ointments, and other transdermal dosage forms. Those compositions may be produced by a conventional method using generally used additives for formulation such as an excipient, an expander, a binder, a wetting agent, a disintegrant, a surfactant, a lubricant, a dispersant, a buffer, a preservative, a solubilizer, an antiseptic, a flavoring agent, a soothing agent, and a stabilizer. As the excipient, there are given, for example, lactose, fructose, glucose, corn starch, sorbit, and crystalline cellulose. As the disintegrant, there are given, for example, starch, sodium algininate, gelatin, calcium carbonate, calcium citrate, dextrin, magnesium carbonate, and synthetic magnesium silicate. As the binder, there are given, for example, methylcellulose or a salt thereof, ethylcellulose, gum arabic, gelatin, hydroxypropylcellulose, and polyvinylpyrrolidone. As the lubricant, there are given, for example, talc, magnesium stearate, polyethylene glycol, and hydrogenated vegetable oil. As the other additives, there are given, for example, syrup, petrolatum, glycerin, ethanol, propylene glycol, citric acid, sodium chloride, sodium sulfite, and sodium phosphate.

The content of the active ingredient in the composition described above is not particularly limited. In general, the content may be appropriately selected depending on the form of the composition, and is generally 10 to 95% by weight, preferably about 30 to 80% by weight in the whole composition.

The dosage of the present invention is not particularly limited, and is appropriately determined in consideration of, for example, an administration route and an administration form, age, sex, the type of diseases, and the degree of symptoms. In general, however, the dosage is about 0.02 to 200 mg/kg, preferably about 0.2 to 100 mg/kg per day in terms of an active ingredient. The dosage may be administered in one or several divided doses a day.

### EXAMPLES

Hereinafter, the present invention is specifically described by way of examples.

### [Example 1] Synthesis method for

### 9-O-acetyl-12-azide-12,13-dihydro-13-hydroxyjosamycin 18-dimethylacetal

1.0 g of josamycin was dissolved in 15 µl of methylene chloride. To the solution were added 178 µl of pyridine, followed by gradual dropwise addition of 114 µl of acetyl chloride. The mixture was stirred at room temperature for 3 hours, and saturated aqueous sodium bicarbonate was then added. 35 ml of methylene chloride were added, and the resultant was washed successively with saturated aqueous sodium bicarbonate and brine. The organic layer was dried over anhydrous sodium sulfate and then filtered. The filtrate was evaporated under reduced pressure. The resultant concentrate was dissolved by adding 3 ml of methanol. To the solution were added 3 ml of methyl orthoformate and 322 mg of PPTS, and the mixture was stirred at 50°C for 33 hours. To the reaction solution were added saturated aqueous sodium bicarbonate, and the mixture was concentrated under reduced pressure and extracted twice with 50 ml of chloroform. The organic layer was washed successively with saturated aqueous sodium bicarbonate and brine. The organic layer was dried over anhydrous sodium sulfate and then filtered.

The filtrate was concentrated under reduced pressure. The resultant reaction product was dissolved by adding 12 ml of chloroform. To the solution were added 982 mg of 3-chloroperbenzoic acid, and the mixture was subjected to a reaction at room temperature for 14 hours. To the reaction solution were added 50 ml of ethanol, followed by gradual dropwise addition of 18 ml of a 5% sodium dithionite aqueous solution under cooling with ice. The mixture was stirred for 1 hour and then concentrated under reduced pressure. To the residue was added saturated aqueous sodium bicarbonate, and the resultant was extracted twice with 50 ml of chloroform. The organic layer was washed successively with 100 ml of saturated aqueous sodium bicarbonate and 100 ml of brine. The organic layer was dried over anhydrous sodium sulfate and then filtered. The filtrate was concentrated under reduced pressure. The resultant residue was passed through silica gel column chromatography (chloroform-methanol (50:1)) to afford crude 9-O-acetyl-12,13-dihydro-13-hydroxy-epoxyjosamycin 18-dimethylacetal.

280 mg of the compound obtained as described above were dissolved by adding 7.0 ml of ethanol-water (8:1) to the compound. To the solution were added 30 mg of ammonium chloride and 70 mg of sodium azide, and the mixture was stirred at 80°C for 24 hours. To the reaction solution were added 10 ml of water, and the mixture was concentrated under reduced pressure. Then, to the residue were added 50 ml of water, and the resultant was extracted with 100 ml of chloroform. The organic layer was washed successively with saturated aqueous sodium bicarbonate and brine. The organic layer was dried over anhydrous sodium sulfate and then filtered. The filtrate was concentrated under reduced pressure. The resultant residue was purified by silica gel column chromatography (chloroform-methanol-aqueous ammonia (90:1:0.1→80:1:0.1)) to afford 560 mg of a compound.

Physical and chemical properties of this compound
(1) Mass spectrum (ESI): m/z 974 (M+H)⁺
(2) ¹H NMR spectrum (400 MHz, CDCl₃) δ (ppm): 0.94 (d, 3-H), 0.96 (d, 3-H), 1.12 (s, 3H), 1.13 (d, 3H), 1.30 (d, 3H), 1.50-1.90 (m, 3H), 2.04 (s, 3H), 2.10 (s, 3H), 2.50 (br s, 6H), 2.61 (dd, 1-H), 2.85 (dd, 1H), 3.16 (s, 3H), 3.27 (s, 3H), 3.53 (s, 3H), 3.57 (dd, 2'-H), 3.93 (br dd, 1H), 4.00 (br d, 1H), 4.25 (br s, 1H), 4.40-4.65 (m, 4H), 5.05-5.15 (m, 3H), 5.32 (br s, 1H), 5.78 (br s, 2H).

### [Example 2] Synthesis method for

### 9-O-acetyl-12-amino-12,13-dihydro-13-hydroxyjosamycin 18-dimethylacetal

280 mg of the compound of Example 1 were dissolved by adding 4 ml of acetonitrile to the compound. To the solution were added 63 mg of triphenylphosphine, and the mixture was stirred at room temperature for 23 hours. To the reaction solution was added 1 ml of water, and the mixture was stirred for an additional 1 hour. The reaction solution was then concentrated under reduced pressure. The resultant residue was purified by silica gel column chromatography (chloroform-methanol-aqueous ammonia (30:1:0.1)) to afford 180 mg of a compound.

Physical and chemical properties of this compound
(1) Mass spectrum (ESI): m/z 948 (M+H)⁺
(2) ¹H NMR spectrum (400 MHz, CDCl₃) δ (ppm): 0.96 (d, 3H), 1.10 (s, 3H), 1.13 (d, 3H), 1.27 (d, 3H), 1.29 (d, 3H), 1.50-1.90 (m, 5H), 2.02 (s, 3H), 2.11 (s, 3H), 2.30 (d, 2H), 2.50 (s, 6H), 2.64 (dd, 1H), 2.88 (dd, 1H), 3.17 (s, 3H), 3.28 (s, 3H), 3.53 (s, 3H), 3.57 (dd, 1H), 3.69 (br s, 1H), 3.78 (m, 1H), 4.01 (br d, 1H), 4.45 (dq, 1H), 4.53 (d, 1H), 4.61 (d, 1H), 5.06 (d, 1H), 5.15 (m, 2H), 5.28 (dd, 9-H), 5.64 (dd, 1H), 5.82 (dd, 1H).

### [Example 3] Synthesis method for

### 9-O-acetyl-12,13-dihydro-13-hydroxy-12-(N-methyl-N-(3-(quinolin-4-yl)propyl)aminoj osamycin 18-dimethylacetal

180 mg of the compound of Example 2 were dissolved by adding 2 ml of methanol to the compound. To the solution were added 38 mg of 3-(quinolin-4-yl)propylcarbaldehyde and 93 µl of acetic acid, and the mixture was stirred at room temperature for 30 minutes. To the reaction solution were added 26 mg of sodium cyanoborohydride, and the mixture was stirred for an additional 1 hour. Then, to the reaction solution were added 10 ml of saturated aqueous sodium bicarbonate, and the resultant was extracted twice with 20 ml of ethyl acetate. The organic layer was washed successively with 20 ml of saturated aqueous sodium bicarbonate and 20 ml of brine. The resultant organic layer was dried over anhydrous sodium sulfate and then filtered. The filtrate was concentrated under reduced pressure. The resultant residue was purified by silica gel column chromatography (chloroform-methanol-aqueous ammonia (50:1:0.1)) to afford 85 mg of 9-O-acetyl-12,13-dihydro-13-hydroxy-12-(N-(3-(quinolin-4-yl)propyl)aminojosamycin 18-dimethylacetal.

85 mg of the compound were dissolved by adding 2 ml of methanol to the compound. To the solution were added 92 µl of a 37% formaldehyde solution, 130 µl of acetic acid, and 48 mg of sodium cyanoborohydride, and the mixture was stirred for 45 minutes under cooling with ice. To the reaction solution were added 20 ml of saturated aqueous sodium bicarbonate, and the mixture was extracted with 40 ml of ethyl acetate. The organic layer was washed successively with 20 ml of saturated aqueous sodium bicarbonate and 20 ml of brine. The organic layer was dried over anhydrous sodium sulfate and then filtered. The filtrate was concentrated under reduced pressure to afford 86 mg of a crude compound.

Physical and chemical properties of this compound
Mass spectrum (ESI): m/z 1,132 (M+H)⁺
¹H NMR spectrum (400 MHz, CDCl₃) δ (ppm): 0.92 (d, 3H), 0.95 (d, 3H), 1.11 (s, 3H), 1.12 (d, 3H), 1.15 (d, 3H), 1.26 (d, 3H), 2.03 (s, 3H), 2.05 (s, 3H), 2.22 (s, 3H), 2.27 (d, 1H), 2.50 (s, 6H), 2.65 (m, 2H), 3.15 (s, 3H), 3.27 (s, 3H), 3.53 (s, 3H), 3.57 (dd, 1H), 3.96 (m, 2H), 4.50 (m, 3H), 4.60 (d, 1H), 5.00 (m, 1H), 5.06 (d, 1H), 5.15 (br s, 1H), 5.37 (br s, 1H), 5.72 (br dd, 1H), 5.81 (br d, 1H), 7.23 (d, 1H), 7.55 (dd, 1H), 7.68 (dd, 1H), 8.02 (d, 1H), 8.08 (d, 1H), 8.78 (d, 1H).

### [Example 4] Production method for

### 9-O-acetyl-4'-demycarosyl-12,13-dihydro-13-hydroxy-12-(N-methyl-N-(3-(quinolin-4-yl)propyl)aminojosamycin (compound of formula (I))

86 mg of the crude compound of Example 3 were dissolved by adding 1 ml of acetonitrile to the crude compound. To the solution was added 1 ml of water. 73 µl of difluoroacetic acid were added, and the mixture was stirred at 40°C for 60 hours. To the reaction solution were added 20 ml of saturated aqueous sodium bicarbonate, and the resultant was extracted with 40 ml of ethyl acetate. The organic layer was washed successively with 20 ml of saturated aqueous sodium bicarbonate and 20 ml of brine. The organic layer was dried over anhydrous sodium sulfate and then filtered. The filtrate was concentrated under reduced pressure. The resultant residue was purified by preparative TLC (chloroform-methanol-aqueous ammonia (10:1:0.1)) to afford 26 mg of the title compound.

Physical and chemical properties of this compound
(1) Mass spectrum (ESI): m/z 858(M+H)⁺
(2) ¹H NMR spectrum (400 MHz, CDCl₃) δ (ppm): 0.88 (d, 3H), 1.18 (d, 3H), 1.21 (d, 3H), 1.53 (m, 2H), 2.05 (s, 3H), 2.24 (s, 3H), 2.33 (dd, 1H), 2.50 (s, 6H), 3.05 (m, 2H), 3.18 (br t, 1H), 3.26 (dq, 1H), 3.36 (br d, 1H), 3.46 (dd, 1H), 3.53 (s, 3H), 3.97 (m, 2H), 4.45 (d, 1H), 5.00 (ddq, 1H), 5.15 (brs, 1H), 5.23 (br t, 1H), 5.75 (dd, 1H), 5.84 (br d, 1H), 7.23 (d, 1H), 7.54 (dd, 1H), 7.68 (dd, 1H), 8.02 (d, 1H), 8.08 (d, 1H), 8.78 (d, 1H), 9.67 (s, 1H).

### [Example 5] Antibacterial activity test

The compound obtained in the present invention was measured for its in vitro antibacterial activity by a broth microdilution method in accordance with a CLSI method (formerly NCCLS method, M31-A2) (Performance Standards for Antimicrobial Disk and Dilution Susceptibility Tests for Bacteria Isolated from Animals; Approved Standard-Second Edition NCCLS M31-A2 Vol. 22 No. 6 2002). A medium composition used in the measurement is shown below.

A test drug solution in which a test drug was dissolved at 1,280 µg/mL in ethanol was diluted 10-fold with the liquid medium described above. The resultant test drug solution was further subjected to two-step dilution with the liquid medium described above to prepare a test drug solution at each concentration level. The thus prepared test drug solution at each concentration level was dispensed into a 96-well microplate at 100 µL/well, and a test bacterial strain was inoculated at about 5×10⁴ CFU/well.

After having been cultured in the presence of 5% CO₂ at 37°C for 20 to 24 hours, the test bacterial strain was visually observed for the presence or absence of its growth. A minimum drug concentration completely inhibiting the growth of the test bacterial strain was defined as a minimum inhibitory concentration (hereinafter, referred to as MIC).

**Liquid medium**

| | |
|---|---|
| BBL Mueller Hinton II broth (Nippon Becton Dickinson Company, Ltd.) | 22.0 g |
| Lysed horse blood* | 20 mL |
| NAD (Wako Pure Chemical Industries, Ltd.) | 0.2 g |
| Purified water | 1,000 mL |

| | |
|---|---|
| *Lysed horse blood | |

| | |
|---|---|
| Saponin (Kanto Chemical Co., Inc.) | 2.0 g |
| Purified water | 10 mL |
| Defibrinated horse blood (Japan Lamb) | 100 mL |

Saponin was dissolved with purified water. The solution was sterilized and then added to defibrinated horse blood.

MICs (µg/ml) of compound of formula (I) and existing animal antibacterial agents

**TABLE 1**

| Compound Species | Strain | TS | AIV | TMS | TLM | JM | Compound of formula (I) |
|---|---|---|---|---|---|---|---|
| *P. multocida* (bovine) | 11272 | 64 | 128 | 32 | 16 | 16 | 4 |
| | 11325-1 | 16 | 128 | 16 | 4 | 8 | 2 |
| | 11787-24 | 32 | 128 | 16 | 8 | 32 | 4 |
| (swine) | ATCC43019 | 64 | 128 | 32 | 16 | 64 | 4 |
| | 11587-3 | 64 | 256 | 16 | 16 | 32 | 4 |
| | 11745-15 | 64 | 256 | 16 | 8 | 32 | 4 |
| | ATCC43137 | 64 | 256 | 16 | 16 | 32 | 4 |
| *A. pleuropneumoniae* | 11796-1 | 32 | 32 | 16 | 16 | 16 | 4 |
| | 11684-7 | 256 | 64 | 128 | 64 | 64 | 32 |
| | 10895-2 | 16 | 32 | 16 | 16 | 32 | 4 |
| | 11804-2 | 32 | 32 | 16 | 16 | 8 | 8 |
| | ATCC27089 | 32 | 32 | 16 | 32 | 16 | 4 |
| *M. haemolytica* | 11443 | 16 | 32 | 16 | 16 | 16 | 4 |
| | 11467-2 | 128 | 64 | 8 | 32 | 32 | 4 |
| *H. somni* | 11321 | 1 | 1 | 1 | 1 | 2 | 0.5 |
| | 11423-3 | 4 | 8 | 4 | 1 | 8 | 1 |
| *H. parasuis* | 11510-1 | 4 | 16 | 1 | 4 | 8 | 0.5 |
| | 11640-1 | 8 | 32 | 16 | 8 | 32 | 4 |

Table 1 shows the results of MIC measurement carried out mainly for pathogens of bacterial respiratory tract infections problematic in livestock animals such as cattle and swine. The results revealed that, in simultaneous comparison with tylosin (TS), aivlosin AIV, tilmicosin (TMS), tulathromycin (TLM), and josamycin (JM) used as animal antibacterial agents, the compound of the formula (I) obtained in the present invention exhibited remarkably strong antibacterial activities as compared to the existing drugs.

### INDUSTRIAL APPLICABILITY

The compound of the formula (I) has been found to have strong antibacterial activities against main pathogens of bacterial respiratory tract infections problematic in livestock animals such as cattle and swine as compared to the existing macrolides for animals. This has allowed an extremely useful animal antibacterial agent to be provided.

## Claims

1. A compound, which is represented by the following formula (I), or a pharmacologically acceptable salt thereof.

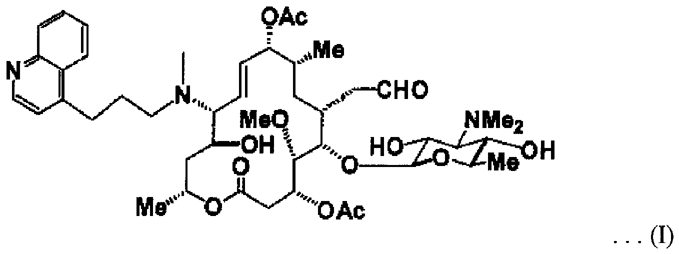

2. A drug, comprising as an active ingredient the compound or the pharmacologically acceptable salt thereof according to claim 1.

3. The drug according to claim 2, which is used as an antibacterial drug.

4. A pharmaceutical composition, comprising as an active ingredient the compound or the pharmacologically acceptable salt thereof according to claim 1, and an additive for formulation.

5. An animal drug, comprising as an active ingredient the compound according to claim 1.

6. An animal antibacterial drug, comprising as an active ingredient the compound according to claim 1.
